# EUROPÄISCHE PATENTSCHRIFT

(11) **EP 2 682 079 B1**
(45) Veröffentlichungstag und Bekanntmachung des Hinweises auf die Patenterteilung: **31.12.2014**
(21) Anmeldenummer: 12175329.7
(22) Anmeldetag: 06.07.2012
(51) Int. Cl.: A61F 9/008

(54) **System zum Einsetzen einer Intracorneallinse**
System for inserting an intracorneal lens
Système d'insertion d'une lentille intracornéenne

(43) Veröffentlichungstag der Anmeldung: 08.01.2014
(73) Patentinhaber: Neoptics AG, 6331 Hünenberg (CH)
(72) Erfinder: Broers, Holger, 266270 Uplengen-Spols (DE); Berner, Werner, 5018 Erlinsbach (DE)
(74) Vertreter: Hepp Wenger Ryffel AG

(56) Entgegenhaltungen:
- WO-A2-03/002047
- WO-A2-2008/072092
- US-A1- 2003 208 189
- US-A1- 2009 247 997

## Beschreibung

Die vorliegende Erfindung betrifft ein System zum Einsetzen einer Intracorneallinse in ein Auge, sowie ein Verfahren und ein Software-Produkt zum Betreiben eines derartigen Systems.

Intracorneallinsen werden zur Korrektur von Sehschwächen herangezogen. Im Gegensatz zu Kontaktlinsen, welche auf der Augenoberfläche aufgebracht sind, und zu Intraocularlinsen, welche in eine Augenkammer implantiert werden, setzt man Intracorneallinsen in eine in der Hornhaut (Cornea) geschaffene Tasche ein. Zur Einführung der Linsen in diese Taschen wird ein Linseneinführungsgerät (Applikator) verwendet.

Intracorneallinsen unterscheiden sich signifikant von Kontaktlinsen oder Intraocularlinsen, beispielsweise in ihrer Grösse, dem Fehlen von bei Intraocularlinsen erforderlichen Halteelementen (haptischen Elementen) sowie in ihren optischen Eigenschaften. Intracorneallinsen sind aus dem Stand der Technik bekannt. Beispielhaft sei auf die WO 2009/075685, die US-5,628,794, die US-5,123,921 oder die EP-1 001 720 B1 verwiesen.

Das Implantieren einer Intracorneallinse erfolgt mit Hilfe eines mehrstufigen Prozesses. Um eine korrekte Ausrichtung der einzusetzenden Intracorneallinse auf einer vorab definierten Achse, vorzugsweise der Sehachse des Auges, zu gewährleisten, wird die Position der Sehachse zunächst durch Einleuchten von Licht in das Auge und anschliessendem Anbringen einer Markierung mit verträglicher Farbe direkt auf der Hornhaut (Cornea) des Auges bestimmt. Anschliessend wird mit Hilfe eines Schneideinstruments, beispielsweise einem Mikrokeratom oder einem Laser, vorzugsweise einem Femtosekundenlaser, anhand der Markierung die Tasche zur Aufnahme der Linse in die Cornea geschnitten. Die Linse wird nun mit einem entsprechenden Instrument in die Tasche in der Cornea eingesetzt. Die korrekte Positionierung der Intracorneallinse in der Tasche wird überprüft und bei Bedarf mit Hilfe eines entsprechenden Instruments nachjustiert.

Die Durchführung sämtlicher vorhergehender Schritte mit höchstmöglicher Präzisierung ist eine erhebliche Herausforderung, welche bis heute noch nicht zufriedenstellend gelöst wurde.

Aus der WO 2011/047076 A1 ist eine Vorrichtung zur Bestimmung der Position der Sehachse beschrieben. Hierbei wird die Sehachse des Auges anhand einer Bezugseinheit (beispielsweise einer Serie konzentrischer Ringe) ausgerichtet und ihre Position mit beispielsweise Tinte auf der Cornea markiert. In diesem Dokument wird somit lediglich eine gewisse Optimierung eines Teilschritts beschrieben.

In der WO 03/053228 A1 ist eine Vorrichtung zur Ablation der Cornea eines Auges beschrieben. Die Steuereinheit eines Lasers wird mit Hilfe einer Bildverarbeitungseinheit kontrolliert. Es werden zwei Bilder des Auges unter verschiedenen Wellenlängen aufgenommen, verarbeitet und mit einem Referenzbild verglichen, welches beispielsweise in anderer Kopfposition des Patienten aufgenommen wurde. Die Aufnahme von zwei Bildern unter unterschiedlicher Lichteinstrahlung dient zur besseren Identifizierung von Verschiebungen der Augenposition gegenüber dem Referenzbild. Diese Vorrichtung ist jedoch nicht zum Implantieren von Intracorneallinsen in Taschen der Cornea geeignet.

Von der Firma SensoMotoric Instruments (SMI, www.surgery-guidance.com) ist ein System bekannt, bei welcher das Auge auf einer zur WO 03/053228 A1 analogen Weise aufgenommen wird. Das Abbild des Auges wird anschliessend in ein Operationsmikroskop eingebracht, um den Chirurg beim Eingriff zu unterstützen. Auch dieses System bietet noch keine vollständige Unterstützung bei der Implantation einer Intracorneallinse in eine Hornhauttasche an.

Es war die Aufgabe der vorliegenden Erfindung, ein System zur verbesserten Implantation einer Intracorneallinse in eine Hornhauttasche bereitzustellen.

Die Aufgabe wird erfindungsgemäss gelöst durch ein System und ein Verfahren zum Betreiben dieses Systems gemäss den unabhängigen Ansprüchen.

Im Einzelnen betrifft die vorliegende Erfindung ein System zum Einsetzen einer Intracorneallinse in ein Auge, umfassend
a) eine Aufnahmeeinheit zum Erstellen mindestens einer Aufnahme des Auges, umfassend eine Bildaufnahmevorrichtung, eine Vorrichtung zur Fixierung der Augenhöhle und gegebenenfalls eine Bezugseinheit, beispielsweise eine zentral auf einem Objektiv der Bildaufnahmevorrichtung angeordnete Lichtquelle, welche vom Auge während des Erstellens der Aufnahme fixierbar ist;
b) eine Bildverarbeitungseinheit, mit welcher das mindestens eine von der Aufnahmeeinheit aufgenommene Abbild des Auges verarbeitbar und ein virtuelles Abbild des Auges mit der gewünschten Position einer in die Cornea zu schneidenden Tasche rechnergesteuert erstellbar ist;
c) eine Lasereinheit zum Schneiden der Tasche in die Cornea des Auges, umfassend eine Steuervorrichtung, mit welcher anhand des von der Bildverarbeitungseinheit erstellten virtuellen Abbilds des Auges die Erstellung der Tasche in der Cornea des Auges durch den Laser rechnergesteuert durchführbar ist;
d) eine Einsetzeinheit zum Einsetzen einer Intracorneallinse in die Tasche in der Cornea, umfassend eine optische Vorrichtung, beispielsweise ein Mikroskop, wobei mit der optischen Vorrichtung das manuelle Einsetzen der Intracorneallinse anhand des von der Bildverarbeitungseinheit erstellten virtuellen Abbilds des Auges und gegebenenfalls anhand eines virtuellen Abbilds eines Geräts zum Einführen der Intracorneallinse durch Überlagerung des virtuellen Abbilds des Auges mit dem durch die optische Vorrichtung sichtbaren reellen Bild des Auges kontrollierbar ist.

Erfindungsgemäss wird ein mit grosser Präzision aufgenommenes Abbild eines Auges zu einem geeigneten virtuellen Abbild verarbeitet, welches alle erforderlichen Informationen zur Durchführung der nachfolgenden Schritte der Implantation einer Intracorneallinse in eine Hornhauttasche aufweist. Dieses virtuelle Abbild wird in sämtlichen nachfolgenden Schritten herangezogen, um eine sehr präzise Implantation einer Intracorneallinse in eine Hornhauttasche zu ermöglichen.

Gemäss der vorliegenden Erfindung wird zunächst mit einer Aufnahmeeinheit mindestens eine Aufnahme des zu behandelnden Auges erstellt. Die Aufnahmeeinheit umfasst eine Bildaufnahmevorrichtung wie eine herkömmliche digitale Kamera, die vorzugsweise auf einem Stativ befestigt ist. Weiterhin ist gemäss einer bevorzugten Ausführungsform der vorliegenden Erfindung eine Bezugseinheit vorgesehen, welche vom aufzunehmenden Auge während des Erstellens der Aufnahme fixierbar ist. Hierbei kann es sich beispielsweise um eine zentral auf einem Objektiv der Bildaufnahmevorrichtung angeordnete Lichtquelle wie eine LED handeln. Die zu behandelnde Person wird angewiesen, während der Aufnahme die Bezugeinheit genau zu fixieren. Dies ermöglicht eine präzise Bestimmung der Sehachse des zu behandelnden Auges. Es ist erfindungsgemäss aber auch möglich, die mindestens eine Aufnahme des Auges ohne eine derartige Bezugeinheit durchzuführen. In diesem Fall wird die mindestens eine Aufnahme des Auges von einem geeigneten Computerprogramm ausgewertet, um die erforderlichen Informationen wie die Position der Sehachse des Auges zu erhalten.

Da ein Patient seinen Kopf während einer derartigen Aufnahmeprozedur in der Regel nicht selbständig ruhig genug halten kann, umfasst die erfindungsgemässe Aufnahmeeinheit zusätzlich eine Vorrichtung zur Fixierung der Augenhöhle. Vorzugsweise handelt es sich hierbei um eine Kopf-Fixierungseinheit, wie sie beispielsweise aus der WO 2011/047076 A1 oder dem vorstehend beschriebenen System von SMI bekannt ist. Beispielhaft umfasst die Kopf-Fixierungseinheit ein fixes Gestell, welches mit Hilfe von Befestigungsmitteln wie Klemmen an einem festen Punkt wie einer Tischplatte fixierbar ist. Das Gestell weist eine Kinnauflageeinheit und eine Stirnanlegeeinheit auf, welche derart angeordnet sind, dass die zu behandelnde Person ihr Kinn auf die Kinnauflageeinheit legen und gleichzeitig die Stirn fest gegen die Stirnanlegeeinheit pressen kann. Vorzugsweise sind die beiden Einheiten verstellbar, um die Kopf-Fixierungseinheit an unterschiedliche Gesichtsformen anpassen zu können. Vorzugsweise kann die Kopf-Fixierungseinheit Mittel zur Befestigung eines Kopfes umfassen, beispielsweise Lederbänder, welche um den Kopf geschlungen und mit Verschlüssen fixiert werden können. Es ist erfindungsgemäss nicht erforderlich, aber selbstverständlich möglich, Aufnahmen unter anderen Lichtbedingungen als Tageslicht aufzunehmen.

Erfindungsgemäss besonders bevorzugt wird die mindestens eine Aufnahme des zu behandelnden Bildes mittels einer Ulbricht-Kugel angefertigt. Eine Ulbricht-Kugel umfasst eine Hohlkugel, wobei durch die Oberflächenbeschaffenheit eine diffuse Reflektion von durch eine Lichtquelle in die Kugel eingestrahltem Licht bewirkt wird. Die Lichtquelle befindet sich vorzugsweise im Winkel von 10-40° zu einem Detektor. Die dadurch bereitgestellte indirekte Beleuchtung ist sehr angenehm für die zu behandelnde Person und erleichtert das Ruhighalten des aufzunehmenden Auges. Zudem können mit dieser bevorzugten Ausführungsform effizient Augengeometrien und insbesondere Augenachsen bestimmt werden.

Erfindungsgemäss besonders bevorzugt wird die mindestens eine Aufnahme des zu behandelnden Auges so angefertigt, dass die Sehachse zentrisch zur Eintrittspupille des Auges liegt. Auf diese Weise wird sichergestellt, dass der Fern- und der Nahanteil der Linse nach Einsetzen der Linse in die Hornhauttasche gleich viel Lichtenergie erhält und ein Zusammenziehen der Pupille bei Lichteinfall nicht zu einem Abschneiden der äusseren Zone der Linse führt.

Gemäss der vorliegenden Erfindung werden vorzugsweise mehrere Aufnahmen, beispielsweise 3 bis 5 Aufnahmen des Auges angefertigt, die zusammen von der Bildverarbeitungseinheit ausgewertet und zu einem virtuellen Abbild des Auges verarbeitet werden.

Die mindestens eine angefertigte Aufnahme des zu behandelnden Auges wird in eine Bildverarbeitungseinheit überführt und dort bearbeitet. Die Überführung des Bildes kann mit Hilfe bekannter Datenübertragungsmethoden erfolgen, beispielsweise mit einer direkten Verbindung zwischen Aufnahmeeinheit und Bildverarbeitungseinheit wie beispielweise einem Datenkabel, einer drahtlosen Verbindung(z.B. WLAN, BLUETOOTH) zwischen Aufnahmeeinheit und Bildverarbeitungseinheit, oder mit Hilfe eine Datenspeichers wie einem USB-Stick. Die Aufnahmeeinheit und Bildverarbeitungseinheit müssen hierzu Mittel zur Datenübertragung wie beispielsweise USB-Schnittstellen aufweisen, an welche ein Datenspeicher wie ein USB-Stick angeschlossen werden kann.

Die erfindungsgemässe Bildverarbeitungseinheit weist entsprechende bekannte Hardware-Komponenten auf, welche für eine Bildbearbeitung erforderlich sind, beispielsweise einen herkömmlichen Computer mit Prozessor und Speichermodulen. Die erfindungsgemässe Bildverarbeitungseinheit erstellt ein virtuelles Abbild des zu behandelnden Auges. Als Bezugssystem wird dabei der Limbus des aufgenommenen Auges herangezogen. Ein wesentlicher Aspekt besteht darin, dass die erfindungsgemässe Bildverarbeitungseinheit in das virtuelle Abbild des zu behandelnden Auges die Position der zu schneidenden Hornhauttasche hineinrechnet und beispielsweise in Form eines Kreises darstellt. Hierbei sollte die Bildverarbeitungseinheit vorzugsweise Mittel zur Kompensation eines Parallaxenfehlers aufweisen. Während des Schneidens der Tasche in die Cornea wird das zu behandelnde Auge mit Hilfe eines Applanators oder eines Laserkopfes zu einem gewissen Grad flachgedrückt. Dies kann dazu führen, dass in diesem Zustand die Lage der zu schneidenden Tasche nicht mehr zentrisch zur Pupille angeordnet ist. Die Bildverarbeitungseinheit der vorliegenden Erfindung sollte dann in der Lage sein, diesen sogenannten Parallaxenfehler sowie die durch die Applanation verursachte Deformation der Hornhaut auszugleichen und bei der Projizierung der Taschenposition in das virtuelle Abbild des Auges zu berücksichtigen.

Mittel, d.h. mathematische Methoden oder entsprechende Computerprogramme, zur Kompensation eines Parallaxenfehlers sind grundsätzlich bekannt. Erfindungsgemäss wird die Bildverarbeitungseinheit mit einem Bildbearbeitungsprogramm betrieben, welches zusätzlich mit entsprechenden Programmabschnitten zur Kompensation eines Parallaxenfehlers ausgestattet ist.

Erfindungsgemäss weist das System eine Lasereinheit zum Schneiden der Tasche in die Cornea des Auges auf. Vorzugsweise handelt es sich um einen Femtosekundenlaser, wie er üblicherweise in der Augenheilkunde eingesetzt wird. Geeignete Laser sind beispielsweise in der US 2003/0014042 A1 oder in der WO2008/072092 A1 beschrieben. Die Lasereinheit umfasst eine Steuervorrichtung, mit welcher anhand des von der Bildverarbeitungseinheit erstellten virtuellen Abbilds des Auges die rechnergesteuerte Erstellung der Tasche in der Cornea des Auges durch den Laser durchführbar ist.

Beide zuvor genannten Dokumente lehren weder eine Aufnahme- noch eine Bildverarbeitungseinheit.

Hierfür müssen die Daten des virtuellen Abbilds des Auges aus der Bildverarbeitungseinheit in die Steuervorrichtung des Lasers überführt werden. Die Überführung der Daten kann mit Hilfe bekannter Datenübertragungsmethoden erfolgen, beispielsweise mit einer direkten Verbindung zwischen Bildverarbeitungseinheit und Lasersteuervorrichtung wie einem Datenkabel, einer drahtlosen Verbindung (WLAN, BLUETOOTH) zwischen Bildverarbeitungseinheit und Lasersteuervorrichtung, oder mit Hilfe eine Datenspeichers wie einem USB-Stick. Die Bildverarbeitungseinheit und Lasersteuervorrichtung müssen hierzu Mittel zur Datenübertragung wie beispielsweise USB-Schnittstellen aufweisen, an welche ein Datenspeicher wie ein USB-Stick angeschlossen werden kann.

Das erfindungsgemäss verwendbare Steuerungsprogramm ist in der Lage, den Betrieb des Lasers anhand der Daten aus der erfindungsgemässen Bildverarbeitungseinheit durchzuführen.

Insbesondere werden erfindungsgemäss die Daten betreffend den Durchmesser des Limbus zum exakten Ausrichten des Laserkopfes herangezogen. Neben der vorstehend beschriebenen virtuellen Projektion der Position der Hornhauttasche in das virtuelle Abbild des Auges können weitere Daten des virtuellen Abbilds des Auges zur Positionierung der Hornhauttasche berücksichtigt werden, beispielsweise Merkmale der Iris.

Mit Hilfe der erfindungsgemässen Lasereinheit ist eine sehr präzise Anfertigung einer Tasche in der Cornea eines Auges möglich, weil der Laser im Gegensatz zu herkömmlichen Lasern anhand des vorstehend beschriebenen virtuellen Abbilds des zu behandelnden Auges sehr genau elektronisch gesteuert werden kann.

Nach Erzeugung der Tasche in der Cornea wird mit Hilfe des erfindungsgemässen Systems die Intracorneallinse in die Tasche manuell eingesetzt. Auch hier ist grosse Präzision erforderlich, um die Linse wie vorstehend erwähnt genau zentrisch zur Eintritttspupille zu positionieren.

Das korrekte Einbringen der erfindungsgemässen Linse in eine dafür geschaffene Tasche in der Cornea kann erfindungsgemäss besonders bevorzugt mit einem Applikator durchgeführt werden, wie er in der WO 2011/069907 A1 beschrieben ist.

In der WO 2011/069907 A1 ist ein Applikator beschrieben, der ein Griffstück und eine Pre-Load-Einheit umfasst. Griffstück und Pre-Load-Einheit können miteinander verbunden werden, vorzugsweise mit Hilfe eines verdrehsicheren Bajonett-Verschlusses. Die Pre-Load-Einheit kann vorgängig mit einer Linse bestückt und in einem Aufbewahrungsbehälter steril gelagert werden. Zum Einsetzen der Linse entnimmt der Arzt die Pre-Load-Einheit aus der Aufbewahrungseinheit und verbindet die Pre-Load-Einheit mit dem Griffstück. Anschliessend kann die Linse auf die in der WO 2011/069907 A1 beschriebene Weise in die Hornhauttasche eingeführt werden.

Vorzugsweise wird ein Kit eingesetzt, umfassend eine Aufbewahrungseinheit und eine Pre-Load-Einheit im Innern der Aufbewahrungseinheit, wobei die Aufbewahrungseinheit aus einem wasserdichten Material besteht und mit einem Stopfen wasserdicht verschliessbar ist und die Pre-Load-Einheit mit einer Intracorneallinse bestückt ist. Um die Pre-Load-Einheit über eine längere Zeit steril lagern zu können, wird sie in einer Aufbewahrungseinheit verpackt, welche die Pre-Load-Einheit vor Umwelteinflüssen schützt. Zu diesem Zweck ist die Aufbewahrungseinheit im Innern mit einer Lagerflüssigkeit gefüllt, welche mindestens die in der Kammer der Pre-Load-Einheit befindliche Linse ständig bedeckt. Es kann sich dabei um Wasser handeln; bevorzugt ist aber physiologische Kochsalzlösung (NaCl) als Lagerflüssigkeit.

Die Pre-Load-Einheit ist in der WO 2011/069907 A1 ausführlich beschrieben. Die Pre-Load-Einheit zur Einführung von Linsen in das Auge eines Menschen oder Tieres umfasst
i) ein Gehäuse mit Mitteln zur, vorzugsweise verdrehsicheren, Befestigung der Einheit an einem Griffstück,
ii) ein Linsenaufnahmeteil, welches an oder in dem Gehäuse angeordnet ist und einen aus dem Gehäuse herausstehenden Abschnitt mit vorzugsweise genau zwei getrennten blattartigen Einheiten umfasst, welche zumindest an ihren vom Gehäuse entfernten Enden miteinander in lösbarem Kontakt stehen und dort eine Kammer zur Aufbewahrung einer optischen Linse bilden,
iii) einen Schieber, welcher beweglich im Innern des Gehäuses angeordnet ist und zwischen den blattartigen Einheiten der Linsenaufnahmeeinheit bewegt werden kann.

Nach Entnahme aus der Aufbewahrungseinheit wird die Pre-Load-Einheit auf ein Griffstück aufgesetzt, welches ebenfalls in der WO 2011/069907 A1 ausführlich beschrieben ist. Typischerweise handelt es sich hierbei um ein längliches Rohr mit einer Form, welche ein einfaches Halten des Griffstücks in der Hand gewährleistet. Das Griffstück ist an einem Ende derart ausgebildet, dass die Pre-Load-Einheit und das Griffstück derart miteinander verbunden werden können, dass die Pre-Load-Einheit innerhalb des Griffstücks bewegt werden kann. Hierzu weist das Griffstück einen Durchmesser auf, welcher den Durchmesser des in das Griffstück einzuführenden Teils der Pre-Load-Einheit übersteigt.

Mit Hilfe des vorstehend beschriebenen Applikators der WO 2011/069907 A1 kann eine Intracorneallinse auf einfache Weise in eine Tasche in der Cornea eingeführt werden. Das Einsetzverfahren umfasst die Schritte:
a) Positionieren des vorstehend beschriebenen Applikators an der gewünschten Stelle des Auges in der korrekten Ausrichtung, vorzugsweise in einer Tasche in der menschlichen Cornea, so dass das Zentrum der im Applikator enthaltenen optischen Linse auf der Sehachse des Auges liegt;
b) Vorschieben des Schiebers mittels eines ersten Bedienelements am Griffstück, bis der Schieber in Kontakt mit der Linse kommt ohne diese zu bewegen, wobei gleichzeitig die blattartigen Einheiten des Linsenaufnahmeteils voneinander weg gespreizt werden;
c) Zurückziehen der restlichen Pre-Load-Einheit um einen definierten Betrag mittels eines zweiten Bedienelements am Griffstück unter gleichzeitiger Fixierung des Schiebers, wodurch die Linse aus dem Applikator freigesetzt wird.

Erfindungsgemäss wird das Einbringen einer Intracorneallinse in die Hornhauttasche mit Hilfe des vorstehend beschriebenen Applikators unter Verwendung einer Einsetzeinheit durchgeführt. Diese Einsetzeinheit zum Einsetzen einer Intracorneallinse in die Tasche in der Cornea umfasst eine optische Vorrichtung, beispielsweise ein Mikroskop. Erfindungsgemäss können in der Augenheilkunde üblicherweise verwendete Operationsmikroskope herangezogen werden.

Erfindungsgemäss wird mit der optischen Vorrichtung das Einsetzen der Intracorneallinse anhand des von der Bildverarbeitungseinheit erstellten virtuellen Abbilds des Auges und gegebenenfalls anhand eines virtuellen Abbilds des Geräts zum Einführen der Intracorneallinse gesteuert und kontrolliert. Dies kann beispielsweise mit Hilfe eines Operationsmikroskops realisiert werden, welches eine Einheit umfasst, mit welchem das von der Bildverarbeitungseinheit erstellte virtuelle Abbild des Auges in den Strahlengang des Mikroskops eingeführt werden kann. Derartige Mikroskope sind bekannt. Beispielhaft wird auf Operationsmikroskope der Firma Leica verwiesen.

Die in den Strahlengang des Mikroskops einführbare Einheit muss mit Daten des virtuellen Abbilds versorgt werden, welche die vorstehend beschriebene Bildverarbeitungseinheit erzeugt hat. Die Zuführung der Daten kann wie vorstehend bei den anderen Einheiten beschrieben mit Hilfe bekannter Datenübertragungsmethoden erfolgen, beispielsweise mit einer direkten Verbindung zwischen Bildverarbeitungseinheit und der Einheit für das Mikroskop wie einem Datenkabel, oder einer drahtlosen Verbindung (WLAN, BLUETOOTH) zwischen Bildverarbeitungseinheit und der Einheit für das Mikroskop.

Anhand der Daten des virtuellen Abbilds des zu behandelnden Auges kann mit Hilfe der vorstehend beschriebenen Einheit im Strahlengang des Mikroskops das virtuelle Abbild des Auges mit der Position der Hornhautasche dargestellt werden. Gemäss einer bevorzugten Ausführungsform der vorliegenden Erfindung kann zusätzlich durch die Einheit auch eine virtuelle Darstellung des Geräts zum Einführen der Intracorneallinse, beispielsweise des vorstehend beschriebenen Applikators, im Strahlengang des Mikroskops erzeugt werden. Hierzu ist eine ständige Datenübertragungsverbindung zwischen der Einheit im Mikroskop und der vorstehend beschriebenen Bildverarbeitungsvorrichtung vorteilhaft, damit erforderliche Rechenkapazität durch die Bildverarbeitungsvorrichtung bereitgestellt werden kann. Es ist aber auch möglich, die Einheit oder das Mikroskop mit eigenen entsprechenden Hardwarekomponenten zu versehen, welche Rechenoperationen durchführen können.

Gemäss der vorliegenden Erfindung erfolgt während des Einsetzens der Linse ein "Tracking" der Augenposition. Während des Einsetzvorgangs tritt eine Bewegung des Auges auf. Erfindungsgemäss wird diese Augenbewegung vom System erfasst und das im Strahlengang der optischen Vorrichtung projizierte virtuelle Abbild des Auges derart modifiziert, dass es ständig mit dem durch die optische Vorrichtung tatsächlich beobachten Bild des Auges überlagert ist. Auf diese Weise ist ein präzises Einsetzen der Linse in die Hornhauttasche gewährleistet.

Beispielsweise werden relevanten Augenmerkmale wie die Struktur der Iris ermittelt, wenn das Auge ruhiggestellt ist. Diese relevanten Augenmerkmale werden bei der vorstehend beschriebenen Erstellung des virtuellen Abbilds des Auges berücksichtigt. Kommt es während des Einsetzens der Linse zu einer Augenbewegung, wird unter Berücksichtigung dieser relevanten Augenmerkmale die neue Position des Auges bestimmt. Das virtuelle Abbild des Auges und gegebenenfalls die Projektion eines Applikators können entsprechend nachgeführt (beispielsweise mittels einer FourierTransformation) und mit dem tatsächlich beobachten Bild des Auges zur Deckung gebracht werden.

Die Überlagerung des virtuellen Abbilds des Auges mit dem tatsächlich beobachteten Bild des Auges erfolgt erfindungsgemäss bevorzugt in der optischen Vorrichtung, wie vorstehend beschrieben. Es ist aber erfindungsgemäss auch möglich, die Überlagerung des virtuellen Abbilds des Auges mit dem tatsächlich beobachteten Bild des Auges auf dem Bildschirm eines entsprechenden elektronischen Geräts (z.B. Desktop-Computer, Tablet-PC) zu realisieren. Die das Einsetzen der Linse durchführende Person kontrolliert dann ihre Handlung anhand der Bildschirmanzeige.

Anhand der virtuellen Darstellungen im Strahlengang des Mikroskops wird der Operateur beim Einsetzen der Intracorneallinse in die Hornhauttasche erheblich unterstützt.

Für den Fall, dass eine Intracorneallinse nicht exakt in die Hornhauttasche eingeführt wurde, kann eine nachträgliche Positionierung der der Linse mit Hilfe eines Positioniergeräts durchgeführt werden. Ein erfindungsgemäss verwendbares Positioniergerät ist nachstehend in Fig. 5a und 5b beschrieben.

Auch die Nachjustierung der Position der Intracorneallinse wird vorzugsweise mit der vorstehend beschriebenen optischen Vorrichtung, beispielsweise dem vorstehend beschriebenen Mikroskop, unter Erzeugung eines virtuellen Abbilds des Auges und gegebenenfalls des Positioniergeräts im Strahlengang der optischen Vorrichtung sehr präzise durchgeführt.

Erfindungsgemäss wird zum ersten Mal ein System gelehrt, bei welchem sämtliche Schritte der Implantation einer Intracorneallinse in die Tasche in der Cornea mit Hilfe eines erstellten virtuellen Abbilds des zu behandelnden Auges und gegebenenfalls des Positioniergeräts (beispielsweise dem vorstehend beschriebenen Applikator) durchgeführt werden. Auf diese Weise kann eine erhebliche Steigerung der Präzision des gesamten Prozesses erreicht werden.

Das erfindungsgemässe System umfasst separate Komponenten, die grundsätzlich nicht in räumlicher Nähe zueinander angeordnet sein müssen. Unter "räumlicher Nähe" wird hierbei ein Abstand der Systemkomponenten voneinander von maximal 10 m verstanden. Erfindungsgemäss ist jedoch bevorzugt, dass zumindest die Komponenten b)-d) (d.h. die Bildverarbeitungsvorrichtung, die Lasereinheit und die Einsetzeinheit) in räumlicher Nähe zueinander angeordnet sind, beispielsweise in einem Operationssaal. Erfindungsgemäss weiterhin bevorzugt ist es, dass die Aufnahmeeinheit nicht im Operationssaal angeordnet ist, wenn die Komponenten b)-d) in einem Operationssaal angeordnet sind.

Die vorliegende Erfindung betrifft weiterhin ein Verfahren zum Betreiben eines vorstehend beschriebenen Systems, umfassend die Schritte
a) Steuerung einer Bildaufnahmevorrichtung, um mindestens eine Aufnahme eines Auges zu erstellen, vorzugsweise wenn die Sehachse des Auges auf eine Bezugseinheit, beispielsweise eine zentral auf einem Objektiv der Bildaufnahmevorrichtung angeordnete Lichtquelle, ausgerichtet ist;
b) Verarbeitung der mindestens einen erstellten Aufnahme des Auges in einer Bildverarbeitungseinheit zu einem virtuellen Abbild des Auges, wobei das virtuelle Abbild des Auges die gewünschte Position einer in die Cornea zu schneidenden Tasche aufweist;
c) Steuerung einer Steuervorrichtung einer Lasereinheit anhand des von der Bildverarbeitungseinheit erstellten virtuellen Abbilds des Auges;
d) Bereitstellung des von der Bildverarbeitungseinheit erstellten virtuellen Abbilds des Auges und gegebenenfalls eines virtuellen Abbilds des Geräts zum Einführen der Intracorneallinse in der optischen Vorrichtung der Einsetzeinheit zur Kontrollierung des Einsetzens einer Intracorneallinse in eine Tasche einer Cornea durch Überlagerung des virtuellen Abbilds des Auges mit dem durch die optische Vorrichtung sichtbaren reellen Bild des Auges.

Die vorliegende Erfindung betrifft weiterhin ein Softwareprodukt zum Betreiben eines vorstehend beschriebenen Systems, wobei das Softwareprodukt die folgenden Schritte ausführt:
a) Steuerung einer Bildaufnahmevorrichtung, um mindestens eine Aufnahme eines Auges zu erstellen, vorzugsweise wenn die Sehachse des Auges auf eine Bezugseinheit, beispielsweise eine zentral auf einem Objektiv der Bildaufnahmevorrichtung angeordnete Lichtquelle, ausgerichtet ist;
b) Verarbeitung der mindestens einen erstellten Aufnahme des Auges in einer Bildverarbeitungseinheit zu einem virtuellen Abbild des Auges, wobei das virtuelle Abbild des Auges die gewünschte Position einer in die Cornea zu schneidenden Tasche aufweist;
c) Steuerung einer Steuervorrichtung einer Lasereinheit anhand des von der Bildverarbeitungseinheit erstellten virtuellen Abbilds des Auges;
d) Bereitstellung des von der Bildverarbeitungseinheit erstellten virtuellen Abbilds des Auges und gegebenenfalls eines virtuellen Abbilds des Geräts zum Einführen der Intracorneallinse in der optischen Vorrichtung der Einsetzeinheit zur Kontrollierung des Einsetzens einer Intracorneallinse in eine Tasche einer Cornea durch Überlagerung des virtuellen Abbilds des Auges mit dem durch die optische Vorrichtung sichtbaren reellen Bild des Auges.

Die vorliegende Erfindung wird nachstehend anhand von nicht einschränkenden Zeichnungen und Beispielen näher erläutert. Es zeigen:
- Fig. 1: eine schematische Darstellung des erfindungsgemässen Systems
- Fig. 2: eine Ausführungsform des Applikators gemäss der WO 2011/069907 A1 zur Einführung der erfindungsgemässen Linse in eine Hornhauttasche
- Fig. 3: eine Ausführungsform einer erfindungsgemässen Aufnahmeeinheit basierend auf einer Ulbricht-Kugel
- Fig. 4a: ein erfindungsgemäss verwendbares Gerät zur Positionierung der erfindungsgemässen Intracorneallinse in einer Hornhauttasche
- Fig. 4b: eine vergrösserte Darstellung des Positionierkopfes des Geräts gemäss Fig. 4a

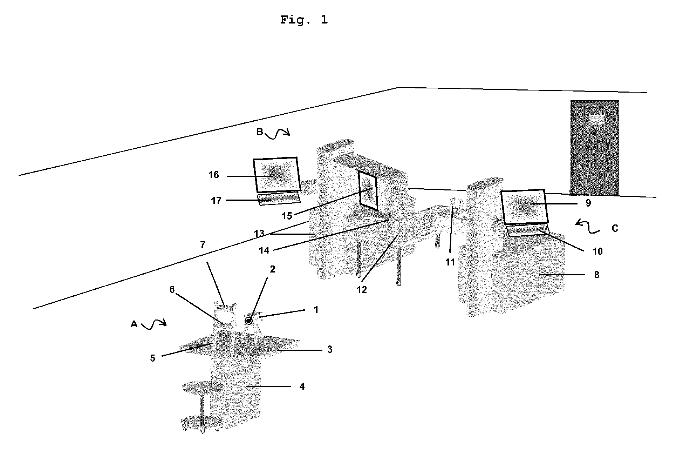

In **Fig. 1** ist eine schematische Darstellung des erfindungsgemässen Systems gezeigt. In einem Raum, beispielsweise einem Operationssaal, sind die einzelnen Komponenten A, B und C angeordnet. Es sei darauf hingewiesen, dass diese räumliche Nähe der Komponenten erfindungsgemäss sehr bevorzugt, aber nicht zwingend erforderlich ist. Wie vorstehend erläutert bestehen Möglichkeiten der Datenübertragung zwischen den einzelnen Komponenten über grössere räumliche Entfernung hinweg. Es ist aber selbstverständlich zweckmässig, die Schritte des Schneidens einer Tasche in die Cornea und des Einsetzens einer Intracorneallinse in diese Tasche in einem Raum durchzuführen. Wie vorstehend bereits ausgeführt ist es aber erfindungsgemäss nicht bevorzugt, dass hierbei die Aufnahmeeinheit im Operationssaal angeordnet ist.

In Fig. 1 ist die Aufnahmeeinheit A zum Erstellen einer Aufnahme des Auges gezeigt. Eine Bildaufnahmevorrichtung 1, hier eine digitale Kamera mit Stativ, ist auf einer Tischplatte 3 aufgestellt. Die Kamera 1 weist ein Objektiv auf, auf welchem zentrisch eine Bezugseinheit 2 angeordnet ist. Es handelt sich in diesem Beispiel um eine Licht-emittierende Diode (LED). Eine zu behandelnde Person kann sich auf den gezeigten Stuhl vor der Aufnahmeeinheit A setzen und ihr Kinn auf eine Kinnablegeeinheit 6 legen, während sie ihre Stirn gleichzeitig gegen eine Stirnanlegeeinheit 7 presst, wodurch die Person ihre Kopfposition für die Aufnahme fixiert. Kinnablegeeinheit 6 und Stirnanlegeeinheit 7 bilden zusammen mit vertikalen Befestigungsstangen eine Vorrichtung zur Fixierung der Augenhöhle 5, hier eine Kopf-Fixierungseinheit.

Es wird nun mit der Kamera 1 mindestens eine Aufnahme des zu behandelnden Auges angefertigt, wobei der Kopf der zu behandelnden Person wie vorstehend beschrieben fixiert ist und die Person mit dem zu behandelnden Auge die Bezugseinheit 2 im Zentrum des Objektivs der Kamera 1 so exakt wie möglich fokussiert. Die Bilddaten werden anschliessend einer Bildverarbeitungseinheit 4 zugeführt, welche im Beispiel gemäss Fig. 1 im Gestell der Tischplatte 3 angeordnet ist. Wie vorstehend beschrieben kann die Datenübermittlung zwischen Aufnahmeeinheit 1 und Bildverarbeitungseinheit 4 beispielsweise mit einer direkten Verbindung wie beispielweise einem Datenkabel, einer drahtlosen Verbindung(z.B. WLAN, BLUETOOTH), oder mit Hilfe eine Datenspeichers wie einem USB-Stick erfolgen. Aufnahmeeinheit 1 und Bildverarbeitungseinheit 4 müssen hierbei Mittel zur Datenübertragung wie USB-Schnittstellen aufweisen, an welche ein Datenspeicher wie ein USB-Stick angeschlossen werden kann.

Es handelt sich bei der Bildverarbeitungseinheit 4 gemäss Fig. 1 um einen Computer mit ausreichender Rechenkapazität, auf welchem das Bildbearbeitungsprogramm vorzugsweise mit Modulen zur Kompensation eines Parallaxenfehlers ausgeführt wird. Mit Hilfe der Bildverarbeitungseinheit 4 wird rechnergesteuert ein virtuelles Abbild des Auges mit der gewünschten Position einer in die Cornea zu schneidenden Tasche erstellt. Interface-Komponenten zur Bedienung der Bildverarbeitungseinheit 4 sind in der Regel vorgesehen, aber in Fig. 1 nicht gezeigt. Bei den Interface-Komponenten kann es sich beispielsweise um einen Monitor zur Anzeige von Daten, Bildern usw. sowie eine Tastatur oder eine Maus zur Daten- und Befehlseingabe handeln.

Nach angefertigter mindestens einer Aufnahme des zu behandelnden Auges begibt sich die zu behandelnde Person auf eine Liege 12. Die Daten betreffend das von der Bildverarbeitungseinheit 4 erstellte virtuelle Abbild des zu behandelnden Auges und der berechneten Position der Linse werden der Steuervorrichtung 13 der Lasereinheit B übergeben, welche sich in einem Gehäuse der Lasereinheit B befindet. Wie vorstehend beschrieben kann die Datenübermittlung zwischen Bildverarbeitungseinheit 4 und Lasereinheit B beispielsweise mit einer direkten Verbindung wie beispielweise einem Datenkabel, einer drahtlosen Verbindung(z.B. WLAN, BLUETOOTH), oder mit Hilfe eine Datenspeichers wie einem USB-Stick erfolgen. Bildverarbeitungseinheit 4 und Lasereinheit B müssen hierbei Mittel zur Datenübertragung wie USB-Schnittstellen aufweisen, an welche ein Datenspeicher wie ein USB-Stick angeschlossen werden kann.

Es handelt sich bei der Steuervorrichtung 13 der Lasereinheit B gemäss Fig. 1 um einen Computer mit ausreichender Rechenkapazität, auf welchem das Steuerprogramm des Lasers ausgeführt wird. Die Steuervorrichtung kann mit üblichen Interface-Komponenten bedient werden. In Fig. 1 ist beispielsweise ein Monitor 16 zur Anzeige von Daten, Bildern usw. sowie eine Tastatur 17 zur Daten- und Befehlseingabe gezeigt. Ein weiterer Monitor 15 kann in der Nähe der Liege 12 angeordnet sein. Der eigentliche Laserkopf 14 ist oberhalb der Liege 12 angeordnet. Vorzugsweise ist die den Laserkopf 14 aufweisende Komponente der Lasereinheit B schwenkbar, um gegebenenfalls Platz oberhalb der Liege 12 zu schaffen. Es handelt sich bei der Lasereinheit B gemäss Fig. 1 um einen Femtosekundenlaser, wie er üblicherweise in der Augenheilkunde eingesetzt wird.

Das Steuerprogramm der Lasereinheit B ist wie vorstehend beschrieben in der Lage, den Vorgang des Schneidens einer Tasche in die Cornea mit Hilfe des Lasers rechnergesteuert anhand des virtuellen Abbilds des zu behandelnden Auges durchzuführen, welches von der Bildverarbeitungseinheit 4 erstellt wurde.

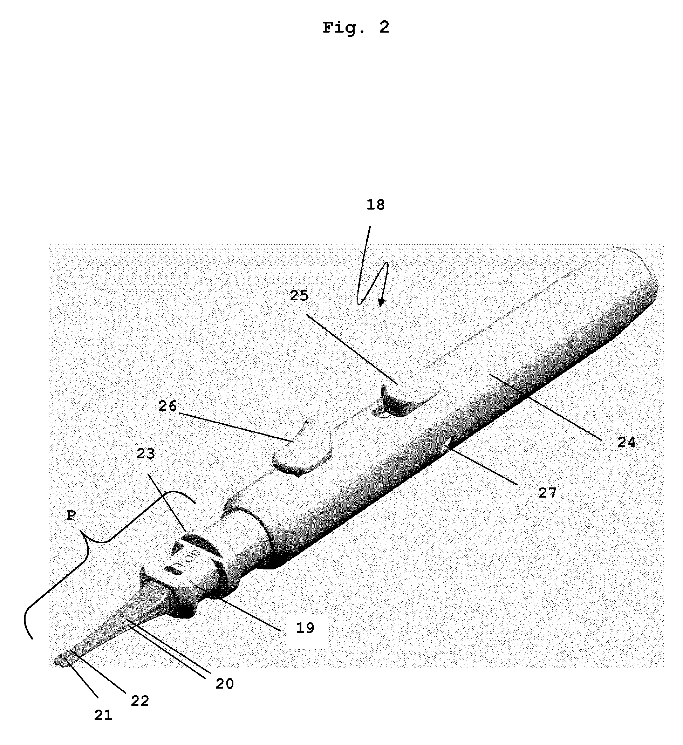

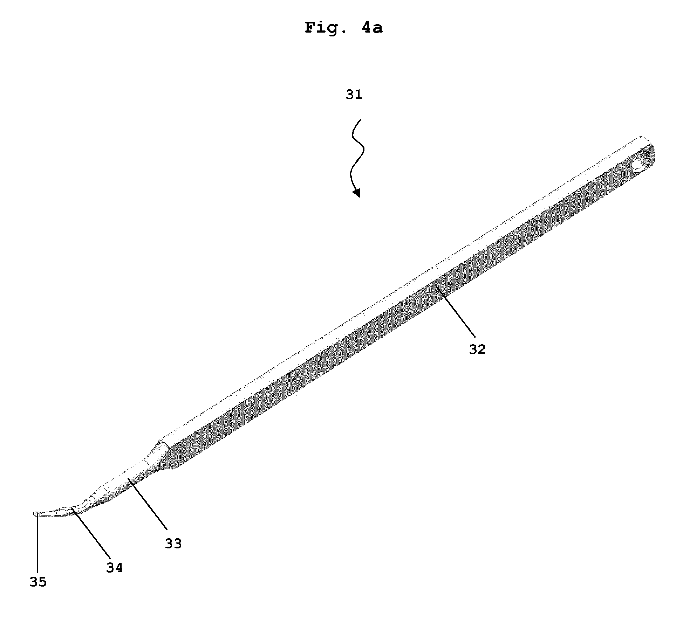

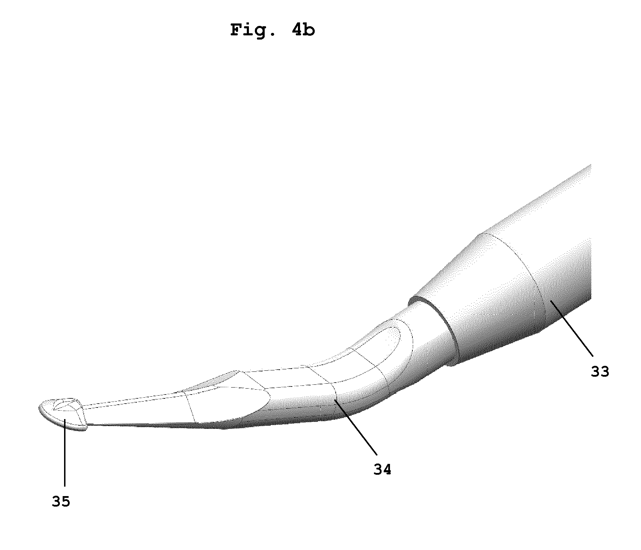

Anschliessend wird eine Intracorneallinse mit Hilfe der Einsetzeinheit C in die angefertigte Tasche in der Cornea manuell eingesetzt. Die Einsetzeinheit C umfasst eine optische Vorrichtung 11. Gemäss Fig. 1 handelt es sich hierbei um ein optisches Mikroskop, wie es üblicherweise in der Augenheilkunde eingesetzt wird. Zum Einsetzen der Linse wird die Liege 12 mit der zu behandelnden Person unterhalb der optischen Vorrichtung 11 platziert. Dies kann entweder durch Bewegung der Liege 12 oder durch ein Schwenken oder Herausziehen der Komponente der Einsetzeinheit C erfolgen, auf welcher sich die optische Vorrichtung 11 befindet. Die das Einsetzen der Linse ausführende Person kontrolliert das Einsetzen der Linse anhand der optischen Vorrichtung 11, während sie das eigentliche Einsetzen der Linse mit Hilfe des vorstehend beschriebenen Applikators durchführt, der nachstehend in den Figuren 2 und 4 noch näher erläutert ist.

Die Kontrolle des Einsetzens der Linse erfolgt erfindungsgemäss ebenfalls anhand des von der Bildverarbeitungseinheit 4 erstellten virtuellen Abbilds des zu behandelnden Auges. Die entsprechenden Daten einer in Fig. 1 nicht gezeigten Einheit im Strahlengang der optischen Vorrichtung 11 der Einsetzeinheit C übergeben. Wie vorstehend beschrieben kann die Datenübermittlung zwischen Bildverarbeitungseinheit 4 und der Einheit im Strahlengang der optischen Vorrichtung beispielsweise mit einer direkten Verbindung wie beispielweise einem Datenkabel, oder einer drahtlosen Verbindung(z.B. WLAN, BLUETOOTH) erfolgen.

In der Ausführungsform gemäss Fig. 1 sind Interface-Einheiten zur Kontrolle und Modifizierung des im Strahlengang der optischen Vorrichtung 11 projizierten virtuellen Abbilds des zu behandelnden Auges vorgesehen. In Fig. 1 ist beispielsweise ein Monitor 9 zur Anzeige von Daten, Bildern usw. sowie eine Tastatur 10 zur Daten- und Befehlseingabe gezeigt. Sollen in der Einsetzeinheit C Rechenoperationen an dem virtuellen Abbild des zu behandelnden Auges durchgeführt werden, kann dies entweder mit der Bildverarbeitungseinheit 4 und Datenübertragung an die Einsetzeinheit C oder mit Hilfe eines Computer mit ausreichender Rechenkapazität erfolgen, welcher beispielsweise im Gehäuse 8 der Einsetzeinheit C angeordnet sein kann. Gemäss der vorliegenden Erfindung erfolgt während des Einsetzens der Linse ein "Tracking" der Augenposition. Während des Einsetzvorgangs tritt eine Bewegung des Auges auf. Erfindungsgemäss wird diese Augenbewegung vom System erfasst und das im Strahlengang der optischen Vorrichtung projizierte virtuelle Abbild es Auges derart modifiziert, dass es ständig mit dem durch die optische Vorrichtung tatsächlich beobachten Bild des Auges überlagert ist. Auf diese weise ist ein präzises Einsetzen der Linse in die Hornhauttasche gewährleistet.

Nach Einsetzen der Linse in die Tasche der Cornea wird mit Hilfe der optischen Vorrichtung 11 der Einsetzeinheit C kontrolliert, ob sich die Linse präzise an der gewünschten Position befindet. Für den Fall, dass eine Intracorneallinse nicht exakt in die Hornhauttasche eingeführt wurde, wird eine nachträgliche Positionierung der der Linse mit Hilfe eines Positioniergeräts durchgeführt werden. Ein erfindungsgemäss verwendbares Positioniergerät ist im Detail nachstehend in Fig. 5a und 5b beschrieben.

Auch die Nachjustierung der Position der Intracorneallinse wird unter Erzeugung eines virtuellen Abbilds des Auges und gegebenenfalls des Positioniergeräts im Strahlengang der optischen Vorrichtung wie vorstehend für den Einsatzvorgang beschrieben sehr präzise durchgeführt.

In **Fig. 2** ist eine Ausführungsform des Applikators 18 aus der WO 2011/069907 A1 gezeigt. In dem Griffstück 24 ist eine Pre-Load-Einheit P beweglich angebracht. Die Pre-Load-Einheit P umfasst ein Gehäuse 19, zwei blattartige Einheiten 20 und einen Stopper 23, welcher das Hineinbewegen der Pre-Load-Einheit P in das Griffstück 24 begrenzt. In den blattartigen Einheiten 20 ist eine durchgehende Öffnung 21 durch das Zentrum der (hier nicht erkennbaren) Kammer zur Aufnahme einer Linse vorhanden. Zudem ist ein weiteres Loch 22 bereitgestellt, welches das Beschicken des Applikators 18 mit einer Linse vereinfacht. Auf dem Griffstück 24 sind zwei Bedienelemente 25 und 26 angebracht, mit deren Hilfe die Pre-Load-Einheit P sowie ein in Fig. 2 nicht sichtbarer Schieber im Innern der Pre-Load-Einheit P und des Griffstücks 24 bewegt werden können. Aus der seitlichen Öffnung 27 ragt ein Stift heraus, welcher an im Griffstück vorhandenen Einlegeteilen befestigt ist und diese im Griffstück 24 fixiert. Die Oberseite der Pre-Load-Einheit P ist deutlich mit dem Wort "TOP" gekennzeichnet.

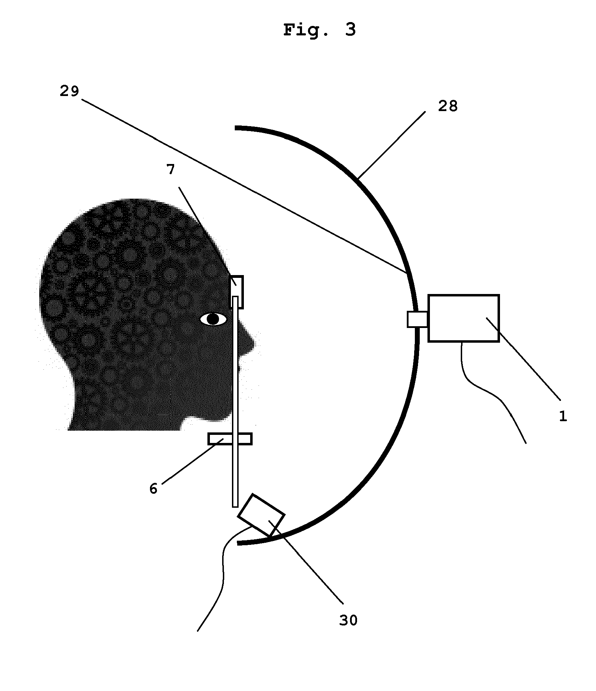

In **Fig. 3** ist eine Ausführungsform einer erfindungsgemässen Aufnahmeeinheit A basierend auf einer Ulbricht-Kugel gezeigt. Der Kopf der zu behandelnden Person ist, wie vorstehend zur Fig. 1 ausgeführt, mit Hilfe einer Kinnablegeeinheit 6 und einer Stirnanlegeeinheit 7 fixiert. Die Fixierung erfolgt dabei so, dass dem abzubildenden Auges eine Kamera 1 mit Objektiv gegenüberliegt. Eine Ulbricht-Kugel 28, welche auf ihrer Innenseite 29 mit einer Schicht aus diffus reflektierendem Material (beispielsweise Polytetrafluorethylen (PTFE, Teflon) beschichtet ist, ist zwischen der Kamera 1 und Kinnablegeeinheit 6 und Stirnanlegeeinheit 7 angeordnet. Licht aus einer Lichtquelle 30 (z.B. eine LED) wird auf die Innenseite 29 der Ulbricht-Kugel 28 gestrahlt und dort diffus reflektiert.

In **Fig. 4a** **und** **4b** ist ein erfindungsgemäss verwendbares Gerät zur Positionierung der erfindungsgemässen Intracorneallinse in einer Hornhauttasche dargestellt. Dieses Positioniergerät kann zum Einsatz kommen, wenn die Intracorneallinse nach Einführung in einer Hornhauttasche noch nicht exakt positioniert ist.

Das Positioniergerät 31 umfasst einen Griff 32 zur Bedienung des Geräts. Mit dem Griff 32 einstückig oder trennbar verbunden ist ein Übergangselement 33. Das Übergangselement 33 weist einen gebogenen Abschnitt 34 auf, welcher vorzugsweise um einen Winkel von etwa 30° von einer von Griff 32 und Übergangselement 33 gebildeten gedachten Geraden weg gebogen ist. Dadurch kann das Positioniergerät 31 von einer Person optimal bestimmungsgemäss bedient werden. Der Abschnitt 34 kann mit dem Übergangselement 33 einstückig oder trennbar verbunden sein. Der Abschnitt 34 verjüngt sich vorzugsweise an dem vom Übergangselement 33 abgewandten Ende, bis es in einen Positionierkopf 35 übergeht. Der Positionierkopf 35 weist vorzugsweise eine halbkreisförmige Form auf und ist mit dem Abschnitt 34 einstückig oder trennbar verbunden.

## Patentansprüche

1. System zum Einsetzen einer Intracorneallinse in ein Auge, umfassend
a) eine Aufnahmeeinheit (A) zum Erstellen mindestens einer Aufnahme des Auges, umfassend eine Bildaufnahmevorrichtung (1), eine Vorrichtung zur Fixierung der Augenhöhle (5) und gegebenenfalls eine Bezugseinheit (2), beispielsweise eine zentral auf einem Objektiv der Bildaufnahmevorrichtung (1) angeordnete Lichtquelle, welche vom Auge während des Erstellens der Aufnahme fixierbar ist;
b) eine Bildverarbeitungseinheit (4), mit welcher das mindestens eine von der Aufnahmeeinheit (A) aufgenommene Abbild des Auges verarbeitbar und ein virtuelles Abbild des Auges mit der gewünschten Position einer in die Cornea zu schneidenden Tasche rechnergesteuert erstellbar ist;
c) eine Lasereinheit (B) zum Schneiden der Tasche in die Cornea des Auges, umfassend eine Steuervorrichtung (13), mit welcher anhand des von der Bildverarbeitungseinheit (4) erstellten virtuellen Abbilds des Auges die Erstellung der Tasche in der Cornea des Auges durch den Laser rechnergesteuert durchführbar ist;
d) eine Einsetzeinheit (C) zum Einsetzen einer Intracorneallinse in die Tasche in der Cornea, umfassend eine optische Vorrichtung (11), beispielsweise ein Mikroskop, wobei mit der optischen Vorrichtung das manuelle Einsetzen der Intracorneallinse anhand des von der Bildverarbeitungseinheit erstellten virtuellen Abbilds des Auges und gegebenenfalls anhand eines virtuellen Abbilds des Geräts zum Einführen der Intracorneallinse durch Überlagerung des virtuellen Abbilds des Auges mit dem durch die optische Vorrichtung sichtbaren reellen Bild des Auges kontrollierbar ist.

2. System nach Anspruch 1, **dadurch gekennzeichnet, dass** die gewünschte Position der in die Cornea zu schneidenden Tasche durch die Bildverarbeitungseinheit (4) anhand des Limbus des Auges als Bezugssystem ausbildbar ist.

3. System nach Anspruch 1 oder 2, **dadurch gekennzeichnet, dass** die Vorrichtung zur Fixierung der Augenhöhle (5) eine Kopf-Fixierungseinheit ist.

4. System nach einem der Ansprüche 1 bis 3, **dadurch gekennzeichnet, dass** die Aufnahmeeinheit (A) eine Ulbricht-Kugel (28) aufweist.

5. System nach einem der Ansprüche 1 bis 4, **dadurch gekennzeichnet, dass** die Bildverarbeitungseinheit (4) Mittel zur Kompensation eines Parallaxenfehlers aufweist.

6. System nach einem der Ansprüche 1 bis 5, **dadurch gekennzeichnet, dass** die Aufnahmeeinheit (A) und die Bildverarbeitungseinheit (4) miteinander durch Mittel zur Datenübertragung verbunden oder verbindbar sind.

7. System nach einem der Ansprüche 1 bis 6, **dadurch gekennzeichnet, dass** die Bildverarbeitungseinheit (4) und die Lasereinheit (B) miteinander durch Mittel zur Datenübertragung verbunden oder verbindbar sind.

8. System nach einem der Ansprüche 1 bis 7, **dadurch gekennzeichnet, dass** es sich bei der optischen Vorrichtung (11) um ein Operationsmikroskop handelt, welches eine Einheit umfasst, mit welcher das von der Bildverarbeitungseinheit (4) erstellte virtuelle Abbild des Auges in den Strahlengang des Mikroskops einführbar ist.

9. System nach Anspruch 8, **dadurch gekennzeichnet, dass** die Einheit direkt mit der Bildverarbeitungseinheit (4) verbunden ist, wobei die direkte Verbindung mit einem Datenkabel erfolgt.

10. System nach einem der Ansprüche 1 bis 9, **dadurch gekennzeichnet, dass** die Einsetzeinheit (C) zusätzlich ein Gerät zum Einführen der Intracorneallinse in die Tasche in der Cornea umfasst.

11. System nach einem der Ansprüche 1 bis 10, **dadurch gekennzeichnet, dass** das System zusätzlich eine Vorrichtung (31) zur Nachjustierung der Position der in die Tasche in der Cornea eingesetzten Intracorneallinse umfasst, wobei die Vorrichtung (31) vorzugsweise einen Griff (32) zur Bedienung des Geräts umfasst, mit welchem ein Übergangselement (33) einstückig oder trennbar verbunden ist, wobei das Übergangselement (33) einen mit dem Übergangselement (33) einstückig oder trennbar verbundenen gebogenen Abschnitt (34) aufweist, welcher vorzugsweise um einen Winkel von etwa 30° von einer von Griff (32) und Übergangselement (33) gebildeten gedachten Geraden weg gebogen ist und in einen Positionierkopf (35) übergeht, wobei der Positionierkopf (35) vorzugsweise eine halbkreisförmige Form aufweist und mit dem Abschnitt (34) einstückig oder trennbar verbunden ist.

12. System nach einem der Ansprüche 1 bis 11, **dadurch gekennzeichnet, dass** zumindest die Komponenten b)-d) in räumlicher Nähe zueinander anordenbar sind, beispielsweise in einem Operationssaal.

13. Verfahren zum Betreiben eines Systems, gemäss einem der Ansprüche 1 bis 12, umfassend die Schritte
a) Steuerung einer Bildaufnahmevorrichtung (1), um mindestens eine Aufnahme eines Auges zu erstellen, vorzugsweise wenn die Sehachse des Auges auf eine Bezugseinheit (2), beispielsweise eine zentral auf einem Objektiv der Bildaufnahmevorrichtung (1) angeordnete Lichtquelle, ausgerichtet ist;
b) Verarbeitung der mindestens einen erstellten Aufnahme des Auges in einer Bildverarbeitungseinheit (4) zu einem virtuellen Abbild des Auges, wobei das virtuelle Abbild des Auges die gewünschte Position einer in die Cornea zu schneidenden Tasche aufweist;
c) Steuerung einer Steuervorrichtung (13) einer Lasereinheit (B) anhand des von der Bildverarbeitungseinheit erstellten virtuellen Abbilds des Auges;
d) Bereitstellung des von der Bildverarbeitungseinheit (4) erstellten virtuellen Abbilds des Auges und gegebenenfalls eines virtuellen Abbilds des Geräts zum Einführen der Intracorneallinse in der optischen Vorrichtung (11) der Einsetzeinheit (C) zur Kontrollierung des Einsetzens einer Intracorneallinse in eine Tasche einer Cornea durch Überlagerung des virtuellen Abbilds des Auges mit dem durch die optische Vorrichtung (11) sichtbaren reellen Bild des Auges.

14. Verfahren nach Anspruch 13, **dadurch gekennzeichnet, dass** die Verarbeitung der erstellten Aufnahme des Auges zu einem virtuellen Abbild des Auges unter Kompensation eines Parallaxenfehlers erfolgt.

15. Softwareprodukt zum Betreiben eines Systems gemäß den Ansprüchen 1-12, wobei das Softwareprodukt die folgenden Schritte ausführt:
a) Steuerung einer Bildaufnahmevorrichtung (1), um mindestens eine Aufnahme eines Auges zu erstellen, vorzugsweise wenn die Sehachse des Auges auf eine Bezugseinheit (2), beispielsweise eine zentral auf einem Objektiv der Bildaufnahmevorrichtung (1) angeordnete Lichtquelle, ausgerichtet ist;
b) Verarbeitung der mindestens einen erstellten Aufnahme des Auges in einer Bildverarbeitungseinheit (4) zu einem virtuellen Abbild des Auges, wobei das virtuelle Abbild des Auges die gewünschte Position einer in die Cornea zu schneidenden Tasche aufweist;
c) Steuerung einer Steuervorrichtung (13) einer Lasereinheit (B) anhand des von der Bildverarbeitungseinheit erstellten virtuellen Abbilds des Auges;
d) Bereitstellung des von der Bildverarbeitungseinheit (4) erstellten virtuellen Abbilds des Auges und gegebenenfalls eines virtuellen Abbilds des Geräts zum Einführen der Intracorneallinse in der optischen Vorrichtung (11) der Einsetzeinheit (C) zur Kontrollierung des Einsetzens einer Intracorneallinse in eine Tasche einer Cornea durch Überlagerung des virtuellen Abbilds des Auges mit dem durch die optische Vorrichtung (11) sichtbaren reellen Bild des Auges.

## Claims

1. System for inserting an intracorneal lens into an eye, comprising
a) a recording unit (A) for producing at least one record of the eye, comprising an image recording device (1), a device for fixing the eye socket (5) and optionally a reference unit (2), for example a light source arranged centrally on a lens of the image recording device (1), which can be fixated on by the eye while the record is produced;
b) an image processing unit (4) by means of which the at least one image of the eye recorded by the recording unit (A) is processable and a virtual image of the eye with the desired position of a pocket to be cut into the cornea is producible under computer control;
c) a laser unit (B) for cutting the pocket into the cornea of the eye, comprising a control device (13) by means of which the production of the pocket in the cornea of the eye by the laser is performable under computer control on the basis of the virtual image of the eye produced by the image processing unit (4);
d) an insertion unit (C) for inserting an intracorneal lens into the pocket in the cornea, comprising an optical device (11), for example a microscope, wherein the optical device allows monitoring of the manual insertion of the intracorneal lens on the basis of the virtual image of the eye produced by the image processing unit and, optionally, on the basis of a virtual image of the instrument for inserting the intracorneal lens by superposing the real image of the eye visible through the optical device on the virtual image of the eye.

2. System according to Claim 1, **characterized in that** the desired position of the pocket to be cut into the cornea is implementable by the image processing unit (4) on the basis of the limbus of the eye as reference system.

3. System according to Claim 1 or 2, **characterized in that** the device for fixing the eye socket (5) is a head fixation unit.

4. System according to one of Claims 1 to 3, **characterized in that** the recording unit (A) comprises an Ulbricht sphere (28).

5. System according to one of Claims 1 to 4, **characterized in that** the image processing unit (4) comprises means for compensation of a parallax error.

6. System according to one of Claims 1 to 5, **characterized in that** the recording unit (A) and the image processing unit (4) are connected or connectable to one another by means for data transmission.

7. System according to one of Claims 1 to 6, **characterized in that** the image processing unit (4) and the laser unit (B) are connected or connectable to one another by means for data transmission.

8. System according to one of Claims 1 to 7, **characterized in that** the optical device (11) is a surgical microscope, which comprises a unit by means of which the virtual image of the eye produced by the image processing unit (4) is insertable into the beam path of the microscope.

9. System according to Claim 8, **characterized in that** the unit is directly connected to the image processing unit (4), wherein the direct connection is brought about by means of a data cable.

10. System according to one of Claims 1 to 9, **characterized in that** the insertion unit (C) additionally comprises an instrument for inserting the intracorneal lens into the pocket in the cornea.

11. System according to one of Claims 1 to 10, **characterized in that** the system additionally comprises a device (31) for readjusting the position of the intracorneal lens inserted into the pocket in the cornea, wherein the device (31) preferably comprises a handle (32) for operating the instrument, to which a transition element (33) is connected in an integral or separable manner, wherein the transition element (33) has a bent section (34) connected to the transition element (33) in an integral or separable manner, which bent section is preferably bent away by an angle of approximately 30° from an imaginary straight line formed by handle (32) and transition element (33) and merges into a positioning head (35), wherein the positioning head (35) preferably has a semicircular-shaped form and is connected to the section (34) in an integral or separable manner.

12. System according to one of Claims 1 to 11, **characterized in that** at least components b)-d) are arrangeable in the spatial vicinity of one another, for example in an operating theatre.

13. Method for operating a system according to one of Claims 1 to 12, comprising the following steps:
a) controlling an image recording device (1) in order to produce at least one recording of an eye, preferably when the visual axis of the eye is aligned with a reference unit (2), for example a light source arranged centrally on a lens of the image recording device (1);
b) processing the at least one produced record of the eye in an image processing unit (4) into a virtual image of the eye, wherein the virtual image of the eye comprises the desired position of a pocket to be cut into the cornea;
c) controlling a control device (13) of a laser unit (B) on the basis of the virtual image of the eye produced by the image processing unit;
d) providing the virtual image of the eye produced by the image processing unit (4) and, optionally, a virtual image of the instrument for inserting the intracorneal lens in the optical device (11) of the insertion unit (C) for monitoring the insertion of an intracorneal lens in a pocket of a cornea by superposing the real image of the eye visible through the optical device (11) on the virtual image of the eye.

14. Method according to Claim 13, **characterized in that** there is compensation of a parallax error when the produced image of the eye is processed into a virtual image of the eye.

15. Software product for operating a system according to Claims 1-12, wherein the software product executes the following steps:
a) controlling an image recording device (1) in order to produce at least one recording of an eye, preferably when the visual axis of the eye is aligned with a reference unit (2), for example a light source arranged centrally on a lens of the image recording device (1);
b) processing the at least one produced record of the eye in an image processing unit (4) into a virtual image of the eye, wherein the virtual image of the eye comprises the desired position of a pocket to be cut into the cornea;
c) controlling a control device (13) of a laser unit (B) on the basis of the virtual image of the eye produced by the image processing unit;
d) providing the virtual image of the eye produced by the image processing unit (4) and, optionally, a virtual image of the instrument for inserting the intracorneal lens in the optical device (11) of the insertion unit (C) for monitoring the insertion of an intracorneal lens in a pocket of a cornea by superposing the real image of the eye visible through the optical device (11) on the virtual image of the eye.

## Revendications

1. Système destiné à insérer une lentille intracornéenne dans un oeil, comprenant
a) une unité d'acquisition (A) destinée à créer au moins une acquisition de l'oeil, comprenant un dispositif d'acquisition d'image (1), un dispositif destiné à fixer l'orbite oculaire (5) et le cas échéant, une unité de référence (2), par exemple une source lumineuse disposés centralement sur un objectif du dispositif d'acquisition d'image (1), qui peut être fixée par l'oeil pendant la création de l'acquisition ;
b) une unité de traitement d'image (4) au moyen de laquelle l'au moins une image de l'oeil acquise par l'unité d'acquisition (A) peut être traitée et une image virtuelle de l'oeil peut être créée de manière commandée par ordinateur avec la position souhaitée d'une poche devant être découpée dans la cornée ;
c) une unité à laser (B) destinée à découper la poche dans la cornée de l'oeil, comprenant un dispositif de commande (13) au moyen duquel la création de la poche dans la cornée de l'oeil peut être effectuée de manière commandée par ordinateur au moyen du laser sur la base de l'image virtuelle de l'oeil créée par l'unité de traitement d'image (4) ;
d) une unité d'insertion (C) destinée à insérer une lentille intracornéenne dans la poche dans la cornée, comprenant un dispositif optique (11), par exemple un microscope, dans lequel l'insertion manuelle de la lentille intra-cornéenne peut être commandée au moyen du dispositif optique sur la base de l'image virtuelle de l'oeil créée par l'unité de traitement d'image et le cas échéant, sur la base d'une image virtuelle de l'appareil destiné à insérer la lentille coréenne par superposition de l'image virtuelle de l'oeil sur l'image réelle de l'oeil visible à travers le dispositif optique.

2. Système selon la revendication 1, **caractérisé en ce que** la position souhaitée de la poche devant être découpée dans la cornée peut être formée par l'unité de traitement d'image (4) sur la base du limbe de l'oeil en tant que système de référence.

3. Système selon la revendication 1 ou 2, **caractérisé en ce que** le dispositif destiné à fixer le globe oculaire (5) est une unité de fixation de la tête.

4. Système selon l'une quelconque des revendications 1 à 3, **caractérisé en ce que** l'unité d'acquisition (A) comprend une sphère intégrante d'Ulbricht (28).

5. Système selon l'une quelconque des revendications 1 à 4, **caractérisé en ce que** l'unité de traitement d'image (4) comprend des moyens destinés à compenser une erreur de parallaxe.

6. Système selon l'une quelconque des revendications 1 à 5, **caractérisé en ce que** l'unité d'acquisition (A) et l'unité de traitement d'image (4) sont ou peuvent être connectées l'une à l'autre par l'intermédiaire de moyens destinés à transmettre des données.

7. Système selon l'une quelconque des revendications 1 à 6, **caractérisé en ce que** l'unité de traitement d'image (4) et l'unité à laser (B) sont ou peuvent être connectées l'une à l'autre par des moyens destinés à transmettre des données.

8. Système selon l'une quelconque des revendications 1 à 7, **caractérisé en ce que** le dispositif optique (11) est un microscope chirurgical qui comprend une unité au moyen de laquelle l'image virtuelle de l'oeil créée par l'unité de traitement d'image (4) peut être introduite sur le chemin optique du microscope.

9. Système selon la revendication 8, **caractérisé en ce que** l'unité est directement connectée à l'unité de traitement d'image (4), la connexion directe étant effectuée au moyen d'un câble de données.

10. Système selon l'une quelconque des revendications 1 à 9, **caractérisé en ce que** l'unité d'insertion (C) comprend en outre un appareil destiné à introduire la lentille intracornéenne dans la poche dans la cornée.

11. Système selon l'une quelconque des revendications 1 à 10, **caractérisé en ce que** le système comprend en outre un dispositif (31) destiné au réajustage de la position de la lentille intracornéenne insérée dans la poche dans la cornée, dans lequel le dispositif (31) comprend de préférence une manette (32) destinée à commander l'appareil, à laquelle un élément de transition (33) est relié de manière solidaire ou séparable, dans lequel l'élément de transition (33) comprend une partie (34) incurvée reliée de manière solidaire ou séparable à l'élément de transition (33), laquelle partie est incurvée de préférence d'un angle d'environ 30° par rapport à une droite imaginaire formée par la manette (32) et l'élément de transition (33) et forme une transition en une tête de positionnement (35), dans lequel la tête de positionnement (35) présente une forme de demi-cercle et est reliée de manière solidaire ou séparable à la partie (34).

12. Système selon l'une quelconque des revendications 1 à 11, **caractérisé en ce qu'**au moins les composants b)-d) peuvent être disposés à proximité spatiale les uns des autres, par exemple dans une salle d'opération.

13. Procéder de mise en fonctionnement d'un système selon l'une quelconque des revendications 1 à 12, comprenant les étapes consistant à
a) commander un dispositif d'acquisition d'image (1) afin de créer au moins une acquisition d'un oeil, de préférence lorsque l'axe de vision de l'oeil est dirigé vers une unité de référence (2), par exemple une source lumineuse disposée centralement sur un objectif du dispositif d'acquisition d'image (1) ;
b) traiter l'au moins une acquisition créée de l'oeil dans une unité de traitement d'image (4) en une image virtuelle de l'oeil, dans lequel l'image virtuelle de l'oeil comprend la position souhaitée d'une poche devant être découpée dans la cornée ;
c) commander un dispositif de commande (13) d'une unité à laser (B) sur la base de l'image virtuelle de l'oeil créée par l'unité de traitement d'image ;
d) fournir l'image virtuelle de l'oeil créée par l'unité de traitement d'image (4) et le cas échéant, une image virtuelle de l'appareil destiné à introduire la lentille intracornéenne dans le dispositif optique (11) de l'unité d'insertion (C) afin de contrôler l'insertion d'une lentille intracornéenne dans une poche d'une cornée par superposition de l'image virtuelle de l'oeil sur l'image réelle de l'oeil visible à travers le dispositif optique (11).

14. Procédé selon la revendication 13, **caractérisé en ce que** le traitement de l'acquisition créée de l'oeil en une image virtuelle de l'oeil s'effectue en compensant une erreur de parallaxe.

15. Produit logiciel destiné à commander un système selon les revendications 1 - 12, dans lequel le produit logiciel exécute les étapes suivantes :
a) commander un dispositif d'acquisition d'image (1) afin de créer au moins une acquisition d'un oeil, de préférence lorsque l'axe de vision de l'oeil est dirigé vers une unité de référence (2), par exemple une source lumineuse disposée centralement sur un objectif du dispositif d'acquisition d'image (1) ;
b) traiter l'au moins une acquisition créée de l'oeil dans une unité de traitement d'image (4) en une image virtuelle de l'oeil, dans lequel l'image virtuelle de l'oeil comprend la position souhaitée d'une poche devant être découpée dans la cornée ;
c) commander un dispositif de commande (13) d'une unité à laser (B) sur la base de l'image virtuelle de l'oeil créée par l'unité de traitement d'image ;
d) fournir l'image virtuelle de l'oeil créée par l'unité de traitement d'image (4) et le cas échéant, une image virtuelle de l'appareil destiné à introduire la lentille intracornéenne dans le dispositif optique (11) de l'unité d'insertion (C) afin de contrôler l'insertion d'une lentille intracornéenne dans une poche d'une cornée par superposition de l'image virtuelle de l'oeil sur l'image réelle de l'oeil visible à travers le dispositif optique (11).
